# EUROPÄISCHE PATENTANMELDUNG

(11) **EP 2 345 403 A2**
(43) Veröffentlichungstag der Anmeldung: **20.07.2011**
(21) Anmeldenummer: 10175528.8
(22) Anmeldetag: 07.09.2010
(51) Int. Cl.: A61K 8/58, A61Q 5/08, A61Q 5/10, A61Q 5/12

(54) **Schonende Haarbehandlungsmittel zur Farbveränderung**

(30) Priorität: 10.12.2009 DE 102009054493
(71) Anmelder: Henkel AG & Co. KGaA, 40589 Düsseldorf (DE)
(72) Erfinder: Janßen, Frank, 41470, Neuss (DE); Wadle, Armin, 40699, Erkrath (DE)

(57) **Zusammenfassung**

Gegenstand der Erfindung ist ein Mittel zur Farbveränderung von keratinischen Fasern, dadurch gekennzeichnet, dass es in einem kosmetischen Träger mindestens eine farbverändernde Komponente und mindestens einen speziellen kationisches Polysiloxan der Formel (I), 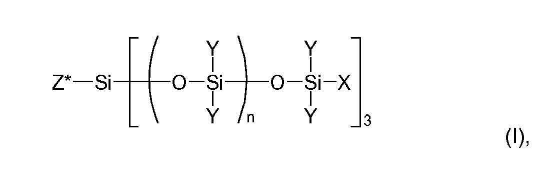 enthält. Die erfindungsgemäßen Mittel eignen sich insbesondere zur Pflege und zur Verbesserung des Oberflächenzustands der Fasern während der oxidativen Haarbehandlung wie Haarfärbung und/oder Haaraufhellung.

## Beschreibung

Gegenstand der Erfindung ist ein Mittel zur Farbveränderung von keratinischen Fasern, welches in einem kosmetischen Träger mindestens eine farbverändernde Komponente sowie mindestens einen bestimmtes kationisches Polysiloxan enthält. Die erfindungsgemäßen Mittel eignen sich insbesondere zur Reduktion der Haarschädigung und zur Verbesserung der Pflege der Haaroberfläche. Weiterhin betrifft die Erfindung ein Verfahren zur Anwendung solcher Mittel auf keratinhaltigen Fasern. Darüber hinaus betrifft die Erfindung die Verwendung des Mittels zur Färbung zur Pflege menschlicher Haare.

Durch Veränderung von Form und Farbe der Haare kann das Erscheinungsbild der Haare sowohl aktuellen Modeströmungen als auch den individuellen Wünschen von einzelnen Personen angepasst werden. Die dazu eingesetzten Mittel sollen neben der gewünschten Färbe und Formleistung möglichst minimale Schädigungen auf dem Haar hervorrufen und insbesondere eine gute Hautverträglichkeit aufweisen.

Zur Bereitstellung farbverändernder kosmetischer Mittel, insbesondere für die Haut oder keratinhaltige Fasern wie beispielsweise menschliche Haare, kennt der Fachmann je nach Anforderungen an die Färbung diverse Färbesysteme.

Für permanente, intensive Färbungen mit entsprechenden Echtheitseigenschaften werden sogenannte Oxidationsfärbemittel verwendet. Solche Färbemittel enthalten üblicherweise Oxidationsfarbstoffvorprodukte, sogenannte Entwicklerkomponenten und Kupplerkomponenten, die unter dem Einfluss von Oxidationsmitteln oder von Luftsauerstoff untereinander die eigentlichen Farbstoffe ausbilden. Die Oxidationsfärbemittel zeichnen sich zwar durch hervorragende, lang anhaltende Färbeergebnisse aus. Für temporäre Färbungen werden üblicherweise Färbe- oder Tönungsmittel verwendet, die als färbende Komponente sogenannte direktziehende Farbstoffe enthalten. Hierbei handelt es sich um Farbstoffmoleküle, die direkt auf das Substrat aufziehen und keinen oxidativen Prozess zur Ausbildung der Farbe benötigen. Schließlich hat ein weiteres Färbeverfahren große Beachtung gefunden, bei dem Vorstufen des natürlichen Haarfarbstoffes Melanin auf das Substrat, z. B. Haare, aufgebracht werden, wobei diese bilden dann im Rahmen oxidativer Prozesse im Haar naturanaloge Farbstoffe ausbilden. Sollen Substrate aufgehellt oder gar gebleicht werden, werden die das Substrat färbenden Farbstoffe meist oxidativ unter Einsatz von entsprechenden Oxidationsmitteln, wie beispielsweise Wasserstoffperoxid, entfärbt.

Oxidative Färbemittel enthalten in der Regel Wasserstoffperoxid als Oxidationsmittel. Dies führt neben dem gewünschten kosmetischen Effekt zu oxidativen Schädigungen an der Haarstruktur und der Haaroberfläche. Darüber hinaus benötigen insbesondere oxidative Farbvermittel-änderungsmittel zumeist einen stark alkalischen pH-Wert, was ebenfalls zu Schädigungen an der Faser führt. Unter diesen harschen Bedingungen können gängige Pflegestoffe ihre Wirkung nur eingeschränkt oder gar nicht mehr entfalten.

Aufgabe der vorliegenden Erfindung ist daher die Bereitstellung kosmetischer Mittel zur Farbveränderung keratinischer Fasern, die gleichzeitig eine verbesserte Pflege der Fasern aufweisen, so dass eine Verbesserung der Faseroberfläche im Hinblick auf Glanz, Geschmeidigkeit und Kämmbarkeit erreicht wird.

In nicht vorhersehbarer Weise konnte nun gefunden werden, dass farbverändernde Mittel, die neben einer farbverändernden Komponente mindestens ein bestimmtes kationisches Polysiloxan enthalten, die oben genannten Nachteile vermeiden. Die eingesetzten Polysiloxane sind als gängige Konditionierstoffe aus der Anmeldung EP1887024 bekannt. Es war jedoch aus dem Stand der Technik nicht absehbar, dass diese Mittel auch unter stark alkalischen und oxidativen Bedingungen eine hervorragende Pflegeleistung entfalten und daher auch in oxidativen Haarbehandlungsmitteln erfolgreich einsetzbar sind.

Ein erster Gegenstand der Erfindung ist daher ein Mittel zur Farbveränderung von keratinischen Fasern, insbesondere menschlichen Haaren, welches in einem kosmetischen Träger mindestens eine farbverändernde Komponente enthält und dadurch gekennzeichnet ist, dass das Mittel zusätzlich mindestens ein kationisches Polysiloxan der Formel (I), 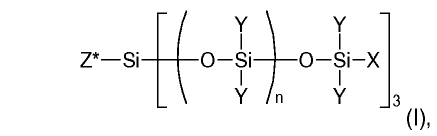 enthält, worin
X für gleiche oder verschiedene Ammoniumfunktionen tragende organische Reste,
Y für gleiche oder verschiedene Reste aus der Gruppe Alkyl, Aryl oder Alkylenaryl mit 1 bis 30 C-Atomen,
Z* für gleiche oder verschiedene Reste aus der Gruppe Alkyl, Aryl, oder Alkylenaryl mit 1 bis 30 C-Atomen,
und n für eine Zahl von 2 bis 200 stehen.

Unter keratinischen Fasern oder auch Keratinfasern sind dabei Pelze, Wolle, Federn und insbesondere menschliche Haare zu verstehen. Obwohl die erfindungsgemäßen Mittel in erster Linie zum Färben von Keratinfasern geeignet sind, steht prinzipiell einer Verwendung auch auf anderen Gebieten nichts entgegen.

Die erfindungsgemäßen Mittel enthalten die Wirkstoffe in einem kosmetischen Träger. Dieser kosmetische Träger ist wässrig, alkoholisch oder wässrig-alkoholisch. Zum Zwecke der Haarfärbung sind solche Träger beispielsweise Cremes, Emulsionen, Gele oder auch tensidhaltige schäumende Lösungen, wie beispielsweise Shampoos, Schaumaerosole oder andere Zubereitungen, die für die Anwendung auf dem Haar geeignet sind. Unter wässrig-alkoholischen Trägern sind im Sinne der vorliegenden Erfindung wasserhaltige Zusammensetzungen, enthaltend 3 bis 70 Gew.-% eines C₁-C₄-Alkohols, bezogen auf das Gesamtgewicht der Anwendungsmischung, insbesondere Ethanol bzw. Isopropanol, zu verstehen. Ein wässriger Träger enthält im Sinne der Erfindung mindestens 30 Gew.-%, insbesondere mindestens 50 Gew.-% Wasser, bezogen auf das Gesamtgewicht der Anwendungsmischung.

Als wesentlichen Inhaltsstoff enthält das erfindungsgemäße Mittel zusätzlich mindestens ein kationisches Polysiloxan der Formel (I). Wie aus Formel (I) deutlich wird, handelt es sich dabei um T-förmige Polysiloxane, die an den drei Enden des T jeweils eine kationische Ladung tragen und damit eine effiziente Bindung zur Haaroberfläche aufbauen können.

Bevorzugt stehen in Formel (I) die Reste Y jeweils unabhängig voneinander für eine Alkyl-Gruppe mit 1 bis 10 C-Atomen, bevorzugt für Methyl, Ethyl oder Isopropyl, wobei Mittel bevorzugt sind, bei denen die Reste Y jeweils für eine Methyl-Gruppe stehen.

Bevorzugt steht in Formel (I) der Rest Z* eine Alkyl-Gruppe mit 1 bis 10 C-Atomen, für eine PhenylGruppe oder eine Benzylgruppe. Bevorzugt steht der Rest Z* für eine Methyl-Gruppe oder eine Phenylgruppe.

Bevorzugt stehen in Formel (I) die Reste X jeweils unabhängig voneinander für eine Gruppe -R1-R2, wobei R1 ein Hydroxygruppen-haltiger Linker und R2 eine Ammoniumgruppen-tragende Einheit darstellt.

Eine Ausführungsform des ersten Erfindungsgegenstands ist daher dadurch gekennzeichnet, dass das Polysiloxan der Formel (I) als Rest X eine Gruppe -R1-R2 enthält, worin R1 für gleiche oder verschiedene zweiwertige Reste, ausgewählt aus den Gruppen (a) bis (d), 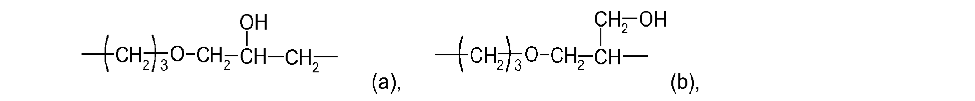 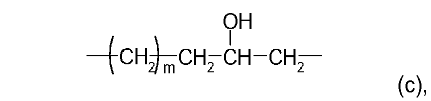 und/oder 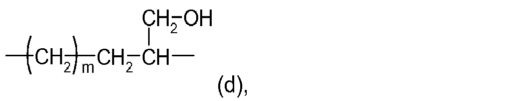 und R2 für eine Ammoniumfunktionen tragende Gruppe, ausgewählt aus den Gruppen (i) bis (iii), 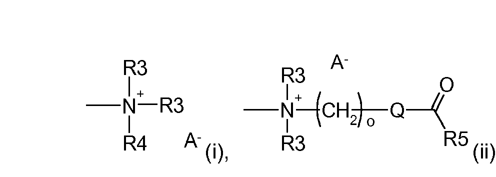 und/oder 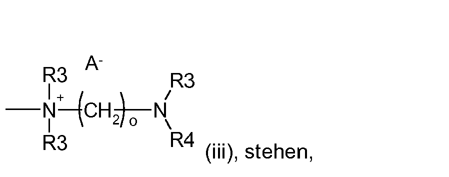 worin
R3, R4, R5 jeweils unabhängig voneinander für Wasserstoff oder einen Alkylrest mit 1 bis 30 C-Atomen,
Q für gleiche oder verschiedene Reste aus der Gruppe O und NR6,
R6 für gleiche oder verschiedene Reste aus Wasserstoff oder Alkyl mit 1 bis 6 C-Atomen,
m für eine Zahl von 2 bis 18 und O für eine Zahl von 2 bis 18 stehen,
wobei A⁻ gleiche oder verschiedene Gegenionen zu den positiven Ladungen an den quaternierten Stickstoffgruppen sind, ausgewählt aus anorganischen oder organischen Anionen der Säuren HA.

Das Gegenion A⁻ zu den positiven Ladungen an den quaternierten Stickstoffgruppen besteht dabei aus dem Anion einer physiologisch verträglichen Säure HA, die besonders bevorzugt aus Essigsäure, Hydroxycarbonsäuren, insbesondere Milchsäure, oder aromatischen Carbonsäuren ausgewählt ist. Weitere bevorzugte Gegenionen stammen aus gängigen Quaternierungsmitteln. Dies sind insbesondere Ethylsulfat, Methylsulfat, Toluolsulfonat, Chlorid und Bromid.

Besonders bevorzugt sind dabei Mittel, bei denen die Reste R1 für den zweiwertigen Rest (a) und R2 die Gruppe (ii) stehen. Eine weitere Ausführungsform des ersten Erfindungsgegenstands ist daher dadurch gekennzeichnet, dass das Polysiloxan der Formel (I) als Rest X eine Gruppe -R1-R2 enthält, worin R1 für 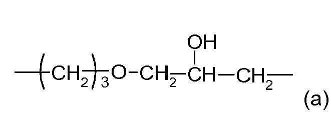 und R2 für 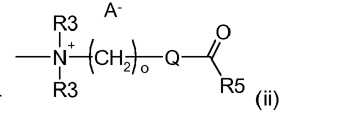 stehen,
worin R3 für einen Alkylrest mit 1 bis 4 C-Atomen, R5 für einen Alkylrest mit 7 bis 23 C-Atomen, o für eine Zahl von 2 bis 5 und Q für die Gruppe NH stehen,
sowie Y für gleiche oder verschiedene Alkyl-Reste mit 1 bis 4 C-Atomen, Z* für gleiche oder verschiedene Reste aus der Gruppe Alkyl, Aryl, oder Alkylenaryl mit 1 bis 30 C-Atomen und n für eine Zahl von 2 bis 200 stehen.

Besonders bevorzugt sind dabei Polysiloxane der Formel (I), worin Z* für eine Methyl- oder PhenylGruppe, Y für jeweils eine Methyl-Gruppe, n jeweils für eine Zahl von 10 bis 120 und X für eine Gruppe -R1-R2 stehen, worin R1 für die Gruppe der Formel (a) und R2 für die Gruppe der Formel (ii) mit R3 für eine Methyl-Gruppe, o für die Zahl 3, Q für NH und R5 für lineare Alkyl-Gruppe mit 9 bis 19 C-Atomen stehen.

Lineare, gesättigte Fettsäure-Reste sind bevorzugte Acyl-Gruppen für die Kombination aus Carbonylgruppe aus (ii) mit dem Rest R5. Solche leiten sich bevorzugt ab von Laurinsäure, Myristinsäure, Palmitinsäure sowie Stearinsäure. Weiterhin kann als Fettsäure ein Gemisch von Fettsäuren unterschiedlicher Kohlenstoffkette eingesetzt werden, insbesondere wenn es sich um Fettsäuren natürlichen Ursprungs handelt. Hierzu wird als Fettsäure ein Fettsäuregemisch, ausgewählt aus Talgfettsäuren oder Palmitinsäure und Stearinsäure, eingesetzt. Besonders bevorzugt leitet sich die Acylgruppe von Laurinsäure ab, d.h. R5 steht für eine lineare Alkyl-Gruppe mit 11 C-Atomen.

Ein erfindungsgemäß besonders geeignetes kationisches Polysiloxan der Formel (I) trägt die INCI-Bezeichnung Silicone Quaternium-22. Dieses Polysiloxan wird als Handelsprodukt, umfassend ca. 65 Gew.-% Polysiloxan und 35 Gew.-% PPG-3 Myristyl Ether, unter dem Handelsnamen Abil T Quat 60 von der Firma Evonik vertrieben.

Eine Ausführungsform der vorliegenden Erfindung ist dadurch gekennzeichnet, das Mittel ein oder mehrere Polysiloxane der Formel (I) in einer Gesamtmenge von 0,001 bis 10 Gew.-%, insbesondere von 0,01 bis 5,0 Gew.-%, jeweils bezogen auf das Gesamtgewicht des anwendungsbereiten Mittels, enthält.

Die erfindungsgemäßen Mittel werden besonders vorteilhaft zur Farbveränderung keratinischer Fasern eingesetzt. Erfindungsgemäß bevorzugte Mittel sind daher dadurch gekennzeichnet, dass das Mittel als farbverändernde Komponente mindestens ein Oxidationsfarbstoffvorprodukt und/oder mindestens einen direktziehenden Farbstoff und/oder mindestens eine Vorstufe naturanaloger Farbstoffe und/oder mindestens Oxofarbstoffvorprodukte und/oder mindestens ein Aufhellmittel enthält.

In einer ersten Ausführungsform werden die farbverändernden Komponenten ausgewählt aus Oxidationsfarbstoffvorprodukten. Eine Ausführungsform des ersten Erfindungsgegenstands ist daher dadurch gekennzeichnet, dass das Mittel als farbverändernde Komponente mindestens ein Oxidationsfarbstoffvorprodukt enthält.

Die Oxidationsfarbstoffvorprodukte werden bevorzugt in einer Menge von 0,005 bis 25 Gew.-%, bevorzugt von 0,05 bis 20 Gew.-% und besonders bevorzugt von 0,1 bis 15 Gew.-%, jeweils bezogen auf das anwendungsbereite Oxidationsfärbemittel, verwendet.

In einer bevorzugten Ausführungsform enthalten die erfindungsgemäßen Mittel mindestens ein Oxidationsfarbstoffvorprodukt vom Entwicklertyp und/oder Kupplertyp. Vorzugsweise enthalten die erfindungsgemäßen Färbemittel mindestens ein Oxidationsfarbstoffvorprodukt vom Entwicklertyp und mindestens ein Oxidationsfarbstoffvorprodukt vom Kupplertyp. Die Entwickler- und Kupplerkomponenten werden üblicherweise in freier Form eingesetzt. Bei Substanzen mit Aminogruppen kann es aber bevorzugt sein, sie in Salzform, insbesondere in Form der Hydrochloride und Hydrobromide oder der Sulfate einzusetzen.

Erfindungsgemäße Entwicklerkomponenten werden ausgewählt aus p-Phenylendiamin, zweikernigen Entwicklerkomponenten, p-Aminophenol, o-Aminophenol, heterocyclischen Entwicklerkomponenten und/oder den Derivaten vorstehender Substanzklassen. Die Entwicklerkomponenten werden bevorzugt in einer Menge von 0,005 bis 20 Gew.-%, vorzugsweise 0,1 bis 15 Gew.-%, jeweils bezogen auf das anwendungsbereite Oxidationsfärbemittel, verwendet.

Bevorzugte p-Phenylendiamine werden ausgewählt aus einer oder mehrerer Verbindungen der Gruppe, die gebildet wird, aus p-Phenylendiamin, p-Toluylendiamin, 2-Chlor-p-phenylendiamin, 2,3-Dimethyl-p-phenylendiamin, 2,6-Dimethyl-p-phenylendiamin, 2,6-Diethyl-p-phenylendiamin, 2,5-Dimethyl-p-phenylendiamin, N,N-Dimethyl-p-phenylendiamin, N,N-Diethyl-p-phenylendiamin, N,N-Dipropyl-p-phenylendiamin, 4-Amino-3-methyl-(N,N-diethyl)anilin, N,N-Bis-(2-hydroxyethyl)-p-phenylendiamin, 4-N,N-Bis-(2-hydroxyethyl)amino-2-methylanilin, 4-N,N-Bis-(2-hydroxyethyl)-amino-2-chloranilin, 2-(2-Hydroxyethyl)-p-phenylendiamin, 2-(1,2-Dihydroxyethyl)-p-phenylendiamin, 2-Fluor-p-phenylendiamin, 2-Isopropyl-p-phenylendiamin, N-(2-Hydroxypropyl)-p-phenylendiamin, 2-Hydroxymethyl-p-phenylendiamin, N,N-Dimethyl-3-methyl-p-phenylendiamin, N-Ethyl-N-2-hydroxyethyl-p-phenylendiamin, N-(2,3-Dihydroxypropyl)-p-phenylendiamin, N-(4'-Aminophenyl)-p-phenylendiamin, N-Phenyl-p-phenylendiamin, 2-(2-Hydroxyethyloxy)-p-phenylendiamin, 2-Methoxymethyl-p-phenylendiamin, 2-(2-Acetylaminoethyloxy)-p-phenylendiamin, N-(2-Methoxyethyl)-p-phenylendiamin, N-(4-Amino-3-methylphenyl)-N-[3-(1H-imidazol-1-yl)propyl]amin, 5,8-Diaminobenzo-1,4-dioxan sowie ihren physiologisch verträglichen Salzen. Erfindungsgemäß besonders bevorzugte p-Phenylendiaminderivate sind ausgewählt aus mindestens einer Verbindung der Gruppe p-Phenylendiamin, p-Toluylendiamin, 2-(2-Hydroxyethyl)-p-phenylendiamin, 2-(1,2-Dihydroxyethyl)-p-phenylendiamin, N,N-Bis-(2-hydroxyethyl)-p-phenylendiamin, N-(4-Amino-3-methylphenyl)-N-[3-(1H-imidazol-1-yl)propyl]amin, 2-Methoxymethyl-p-phenylendiamin sowie deren physiologisch verträglichen Salzen. Es kann erfindungsgemäß weiterhin bevorzugt sein, als Entwicklerkomponente Verbindungen einzusetzen, die mindestens zwei aromatische Kerne enthalten, die mit Amino- und/oder Hydroxylgruppen substituiert sind. Bevorzugte zweikernige Entwicklerkomponenten werden insbesondere aus mindestens einer der folgenden Verbindungen ausgewählt: N,N'-Bis-(2-hydroxyethyl)-N,N'-bis-(4'-aminophenyl)-1,3-diamino-propan-2-ol, N,N'-Bis-(2-hydroxyethyl)-N,N'-bis-(4'-aminophenyl)ethylendiamin, N,N'-Bis-(4'-aminophenyl)tetramethylendiamin, N,N'-Bis-(2-hydroxyethyl)-N,N'-bis-(4'-aminophenyl)tetramethylendiamin, N,N'-Bis-[4-(methylamino)phenyl]tetramethylendiamin, N,N'-Diethyl-N,N'-bis-(4'-amino-3'-methylphenyl)ethylendiamin, Bis-(2-hydroxy-5-aminophenyl)methan, N,N'-Bis-(4'-aminophenyl)-1,4-diazacycloheptan, N,N'-Bis-(2-hydroxy-5-aminobenzyl)piperazin, N-(4'-Aminophenyl)-p-phenylendiamin und 1,10-Bis-(2',5'-diaminophenyl)-1,4,7,10-tetraoxadecan sowie ihre physiologisch verträglichen Salze. Besonders bevorzugte zweikernige Entwicklerkomponenten werden ausgewählt unter N,N'-Bis-(2-hydroxyethyl)-N,N'-bis-(4-aminophenyl)-1,3-diamino-propan-2-ol, Bis-(2-hydroxy-5-aminophenyl)methan, 1,3-Bis-(2,5-diaminophenoxy)propan-2-ol, N,N'-Bis-(4-aminophenyl)-1,4-diazacycloheptan, 1,10-Bis-(2,5-diaminophenyl)-1,4,7,10-tetraoxadecan oder einem deren physiologisch ver-träglichen Salzen. Weiterhin kann es erfindungsgemäß bevorzugt sein, als Entwicklerkomponente ein p-Aminophenolderivat oder eines seiner physiologisch verträglichen Salze einzusetzen. Bevorzugte p-Aminophenole sind insbesondere p-Aminophenol, N-Methyl-p-aminophenol, 4-Amino-3-methylphenol, 4-Amino-3-fluorphenol, 2-Hydroxymethylamino-4-aminophenol, 4-Amino-3-hydroxymethylphenol, 4-Amino-2-(2-hydroxyethoxy)phenol, 4-Amino-2-methylphenol, 4-Amino-2-hydroxymethylphenol, 4-Amino-2-methoxymethylphenol, 4-Amino-2-aminomethylphenol, 4-Amino-2-[(2-hydroxyethyl)aminomethyl]phenol, 4-Amino-2-(1,2-dihydroxyethyl)phenol, 4-Amino-2-fluorphenol, 4-Amino-2-chlorphenol, 4-Amino-2,6-dichlorphenol, 4-Amino-2-(diethylaminomethyl)phenol sowie ihre physiologisch verträglichen Salze. Besonders bevorzugte Verbindungen sind p-Aminophenol, 4-Amino-3-methylphenol, 4-Amino-2-aminomethylphenol, 4-Amino-2-(1,2-dihydroxyethyl)phenol und 4-Amino-2-(diethylaminomethyl)phenol oder einem deren physiologisch verträglichen Salzen. Ferner kann die Entwicklerkomponente ausgewählt sein aus o-Aminophenol und seinen Derivaten, wie 2-Amino-4-methylphenol, 2-Amino-5-methylphenol oder 2-Amino-4-chlorphenol. Weiterhin kann die Entwicklerkomponente ausgewählt sein aus heterocyclischen Entwicklerkomponenten, wie beispielsweise aus Pyrimidinderivaten, Pyrazolderivaten, Pyrazolopyrimidin-Derivaten bzw. ihren physiologisch verträglichen Salzen. Bevorzugte Pyrimidinderivate sind die Verbindungen 2,4,5,6-Tetraaminopyrimidin, 4-Hydroxy-2,5,6-triaminopyrimidin, 2-Hydroxy-4,5,6-triaminopyrimidin, 2-Dimethylamino-4,5,6-triaminopyrimidin, 2,4-Dihydroxy-5,6-diaminopyrimidin und 2,5,6-Triaminopyrimidin. Bevorzugte Pyrazolderivate sind die Verbindungen, die ausgewählt werden unter 4,5-Diamino-1-methylpyrazol, 4,5-Diamino-1-(2-hydroxyethyl)pyrazol, 3,4-Diaminopyrazol, 4,5-Diamino-1-(4'-chlorbenzyl)pyrazol, 4,5-Diamino-1,3-dimethylpyrazol, 4,5-Diamino-3-methyl-1-phenylpyrazol, 4,5-Diamino-1-methyl-3-phenylpyrazol, 4-Amino-1,3-dimethyl-5-hydrazinopyrazol, 1-Benzyl-4,5-diamino-3-methylpyrazol, 4,5-Diamino-3-t-butyl-1-methylpyrazol, 4,5-Diamino-1-t-butyl-3-methylpyrazol, 4,5-Diamino-1-(2-hydroxyethyl)-3-methylpyrazol, 4,5-Diamino-1-ethyl-3-methylpyrazol, 4,5-Diamino-1-ethyl-3-(4'-methoxyphenyl)pyrazol, 4,5-Diamino-1-ethyl-3-hydroxymethylpyrazol, 4,5-Diamino-3-hydroxymethyl-1-methylpyrazol, 4,5-Diamino-3-hydroxymethyl-1-isopropylpyrazol, 4,5-Diamino-3-methyl-1-isopropylpyrazol, 4-Amino-5-(2-aminoethyl)amino-1,3-dimethylpyrazol sowie deren physiologisch verträglichen Salzen. Bevorzugte Pyrazolopyrimidinderivate sind insbesondere die Derivate des Pyrazolo[1,5-a]pyrimidin und dessen tautomeren Formen, sofern ein tautomeres Gleichgewicht besteht. Die Pyrazolo[1,5-a]pyrimidinen sind insbesondere ausgewählt aus Pyrazolo[1,5-a]pyrimidin-3,7-diamin, 2,5-Dimethyl-pyrazolo[1,5-a]pyrimidin-3,7-diamin, Pyrazolo[1,5-a]pyrimidin-3,5-diamin, 2,7-Dimethyl-pyrazolo[1,5-a]pyrimidin-3,5-diamin, 3-Aminopyrazolo[1,5-a]-pyrimidin-7-ol, 3-Aminopyrazolo[1,5-a]pyrimidin-5-ol, 2-(3-Aminopyrazolo[1,5-a]pyrimidin-7-ylamino)ethanol, 2-(7-Aminopyrazolo[1,5-a]pyrimidin-3-ylamino)ethanol, 2-[(3-Aminopyrazolo[1,5-a]-pyrimidin-7-yl)-(2-hydroxyethyl)amino]ethanol, 2-[(7-Aminopyrazolo[1,5-a]pyrimidin-3-yl)-(2-hydroxyethyl)amino]ethanol; 5,6-Dimethylpyrazolo[1,5-a]pyrimidin-3,7-diamin, 2,6-Dimethylpyrazolo[1,5-a]pyrimidin-3,7-diamin, 3-Amino-7-dimethylamino-2,5-dimethylpyrazolo[1,5-a]pyrimidin sowie ihren physiologisch verträglichen Salzen und ihren tautomeren Formen, wenn ein tautomeres Gleichgewicht vorhanden ist.

Besonders bevorzugte Entwicklerkomponenten werden ausgewählt aus mindestens einer Verbindung aus der Gruppe, die gebildet wird aus p-Phenylendiamin, p-Toluylendiamin, 2-(2-Hydroxyethyl)-p-phenylendiamin, 2-(1,2-Dihydroxyethyl)-p-phenylendiamin, N,N-Bis-(2-hydroxyethyl)-p-phenylendiamin, 2-Methoxymethyl-p-phenylendiamin, N-(4-Amino-3-methylphenyl)-N-[3-(1H-imidazol-1-yl)propyl]amin, N,N'-Bis-(2-hydroxyethyl)-N,N'-bis-(4-aminophenyl)-1,3-diamino-propan-2-ol, Bis-(2-hydroxy-5-aminophenyl)methan, 1,3-Bis-(2,5-diaminophenoxy)-propan-2-ol, N,N'-Bis-(4-aminophenyl)-1,4-diazacycloheptan, 1,10-Bis-(2,5-diaminophenyl)-1,4,7,10-tetraoxadecan, p-Aminophenol, 4-Amino-3-methylphenol, 4-Amino-2-aminomethylphenol, 4-Amino-2-(1,2-dihydroxyethyl)phenol und 4-Amino-2-(diethylaminomethyl)phenol, 4,5-Diamino-1-(2-hydroxyethyl)-pyrazol, 2,4,5,6-Tetraaminopyrimidin, 4-Hydroxy-2,5,6-triaminopyrimidin, 2-Hydroxy-4,5,6-triamino-pyrimidin sowie deren physiologisch verträglichen Salzen.

Kupplerkomponenten bilden im Rahmen der oxidativen Färbung allein keine signifikante Färbung aus, sondern benötigen stets die Gegenwart von Entwicklerkomponenten. Daher ist es erfindungsgemäß bevorzugt, dass bei Verwendung mindestens einer Kupplerkomponente zusätzlich mindestens eine Entwicklerkomponente zum Einsatz kommt.

Erfindungsgemäße Kupplerkomponenten werden bevorzugt ausgewählt aus m-Aminophenol, m-Diaminobenzol, o-Diaminobenzol, o-Aminophenol, Naphthalinderivaten mit mindestens einer Hydroxygruppe, Di- beziehungsweise Trihydroxybenzol, Pyridin, Pyrimidin, Monohydroxy- bzw. Monoaminoindol, Monohydroxy- bzw. Monoaminoindolin, Pyrazolon, Benzomorpholin, Chinoxalin und/oder den Derivaten der vorstehenden Substanzklassen. Die Kupplerkomponenten werden bevorzugt in einer Menge von 0,005 bis 20 Gew.-%, vorzugsweise 0,1 bis 15 Gew.-%, jeweils bezogen auf das anwendungsbereite Oxidationsfärbemittel, verwendet.

Die erfindungsgemäß bevorzugten m-Aminophenole bzw. deren Derivate werden ausgewählt aus mindestens einer Verbindung aus der Gruppe, die gebildet wird aus m-Aminophenol, 5-Amino-2-methylphenol, N-Cyclopentyl-3-aminophenol, 3-Amino-2-chlor-6-methylphenol, 2-Hydroxy-4-amino-phenoxyethanol, 2,6-Dimethyl-3-aminophenol, 3-Trifluoroacetylamino-2-chlor-6-methylphenol, 5-Amino-4-chlor-2-methylphenol, 5-Amino-4-methoxy-2-methylphenol, 5-(2'-Hydroxyethyl)amino-2-methylphenol, 3-Diethylaminophenol, N-Cyclopentyl-3-aminophenol, 1,3-Dihydroxy-5-methylamino-benzol, 3-Ethylamino-4-methylphenol, 2,4-Dichlor-3-aminophenol und deren physiologisch verträglichen Salzen. Die erfindungsgemäß bevorzugten m-Diaminobenzole bzw. deren Derivate werden ausgewählt aus mindestens einer Verbindung aus der Gruppe, die gebildet wird aus m-Phenylendiamin, 2-(2,4-Diaminophenoxy)ethanol, 1,3-Bis(2,4-diaminophenoxy)propan, 1-Methoxy-2-amino-4-(2'-hydroxyethylamino)benzol, 1,3-Bis(2,4-diaminophenyl)propan, 2,6-Bis(2'-hydroxyethylamino)-1-methylbenzol, 2-({3-[(2-Hydroxyethyl)amino]-4-methoxy-5-methylphenyl}amino)ethanol, 2-({3-[(2-Hydroxyethyl)amino]-2-methoxy-5-methylphenyl}amino)ethanol, 2-({3-[(2-Hydroxyethyl)amino]-4,5-dimethylphenyl}amino)ethanol, 2-[3-Morpholin-4-ylphenyl)amino]ethanol, 3-Amino-4-(2-methoxy-ethoxy)-5-methylphenylamin, 1-Amino-3-bis-(2'-hydroxyethyl)aminobenzol und deren physiologisch verträglichen Salzen. Die erfindungsgemäß bevorzugten o-Diaminobenzole bzw. deren Derivate werden ausgewählt aus mindestens einer Verbindung aus der Gruppe, die gebildet wird aus 3,4-Diaminobenzoesäure und 2,3-Diamino-1-methylbenzol und deren physiologisch verträglichen Salzen. Bevorzugte Di- beziehungsweise Trihydroxybenzole und deren Derivate werden ausgewählt aus mindestens einer Verbindung der Gruppe, die gebildet wird aus Resorcin, Resorcinmonomethylether, 2-Methylresorcin, 5-Methylresorcin, 2,5-Dimethylresorcin, 2-Chlorresorcin, 4-Chlorresorcin, Pyrogallol und 1,2,4-Trihydroxybenzol. Die erfindungsgemäß bevorzugten Pyridinderivate werden ausgewählt aus mindestens einer Verbindung der Gruppe, die gebildet wird aus 2,6-Dihydroxypyridin, 2-Amino-3-hydroxypyridin, 2-Amino-5-chlor-3-hydroxypyridin, 3-Amino-2-methylamino-6-methoxypyridin, 2,6-Dihydroxy-3,4-dimethylpyridin, 2,6-Dihydroxy-4-methylpyridin, 2,6-Diaminopyridin, 2,3-Diamino-6-methoxypyridin, 3,5-Diamino-2,6-dimethoxypyridin, 3,4-Diaminopyridin, 2-(2-Methoxyethyl)amino-3-amino-6-methoxypyridin, 2-(4'-Methoxyphenyl)amino-3-aminopyridin und deren physiologisch verträglichen Salzen. Bevorzugte Naphthalinderivate mit mindestens einer Hydroxygruppe werden ausgewählt aus mindestens einer Verbindung der Gruppe, die gebildet wird aus 1-Naphthol, 2-Methyl-1-naphthol, 2-Hydroxymethyl-1-naphthol, 2-Hydroxyethyl-1-naphthol, 1,3-Dihydroxynaphthalin, 1,5-Dihydroxynaphthalin, 1,6-Dihydroxynaphthalin, 1,7-Dihydroxynaphthalin, 1,8-Dihydroxynaphthalin, 2,7-Dihydroxynaphthalin und 2,3-Dihydroxynaphthalin. Die erfindungsgemäß bevorzugten Indolderivate werden ausgewählt aus mindestens einer Verbindung der Gruppe, die gebildet wird aus 4-Hydroxyindol, 6-Hydroxyindol und 7-Hydroxyindol und deren physiologisch verträglichen Salzen. Die erfindungsgemäß bevorzugten Indolinderivate werden ausgewählt aus mindestens einer Verbindung der Gruppe, die gebildet wird aus 4-Hydroxyindolin, 6-Hydroxyindolin und 7-Hydroxyindolin und deren physiologisch verträglichen Salzen. Bevorzugte Pyrimidinderivate werden ausgewählt aus mindestens einer Verbindung der Gruppe, die gebildet wird aus 4,6-Diaminopyrimidin, 4-Amino-2,6-dihydroxy-pyrimidin, 2,4-Diamino-6-hydroxypyrimidin, 2,4,6-Trihydroxypyrimidin, 2-Amino-4-methylpyrimidin, 2-Amino-4-hydroxy-6-methylpyrimidin und 4,6-Dihydroxy-2-methylpyrimidin und deren physiologisch verträglichen Salzen.

Erfindungsgemäß besonders bevorzugte Kupplerkomponenten werden ausgewählt unter 3-Aminophenol, 5-Amino-2-methylphenol, 3-Amino-2-chlor-6-methylphenol, 2-Hydroxy-4-aminophenoxy-ethanol, 5-Amino-4-chlor-2-methylphenol, 5-(2-Hydroxyethyl)-amino-2-methylphenol, 2,4-Dichlor-3-aminophenol, 2-Aminophenol, 3-Phenylendiamin, 2-(2,4-Diaminophenoxy)ethanol, 1,3-Bis-(2,4-diaminophenoxy)propan, 1-Methoxy-2-amino-4-(2-hydroxyethylamino)benzol, 1,3-Bis-(2,4-diamino-phenyl)propan, 2,6-Bis-(2'-hydroxyethylamino)-1-methylbenzol, 2-({3-[(2-Hydroxyethyl)amino]-4-methoxy-5-methylphenyl}amino)ethanol, 2-({3-[(2-Hydroxyethyl)amino]-2-methoxy-5-methylphenyl}amino)ethanol, 2-({3-[(2-Hydroxyethyl)amino]-4,5-dimethylphenyl}amino)ethanol, 2-[3-Morpholin-4-ylphenyl)amino]ethanol, 3-Amino-4-(2-methoxyethoxy)-5-methylphenylamin, 1-Amino-3-bis-(2-hydroxyethyl)aminobenzol, Resorcin, 2-Methylresorcin, 4-Chlorresorcin, 1,2,4-Trihydroxybenzol, 2-Amino-3-hydroxypyridin, 3-Amino-2-methylamino-6-methoxypyridin, 2,6-Dihydroxy-3,4-dimethylpyridin, 3,5-Diamino-2,6-dimethoxypyridin, 1-Phenyl-3-methylpyrazol-5-on, 1-Naphthol, 1,5-Dihydroxynaphthalin, 2,7-Dihydroxynaphthalin, 1,7-Dihydroxynaphthalin, 1,8-Dihydroxynaphthalin, 4-Hydroxyindol, 6-Hydroxyindol, 7-Hydroxyindol, 4-Hydroxyindolin, 6-Hydroxyindolin, 7-Hydroxyindolin oder Gemischen dieser Verbindungen oder den physiologisch verträglichen Salzen der vorgenannten Verbindungen.

Dabei werden Entwicklerkomponenten und Kupplerkomponenten im Allgemeinen in etwa molaren Mengen zueinander eingesetzt. Wenn sich auch der molare Einsatz als zweckmäßig erwiesen hat, so ist ein gewisser Überschuss einzelner Oxidationsfarbstoffvorprodukte nicht nachteilig, so dass Entwicklerkomponenten und Kupplerkomponenten in einem Mol-Verhältnis von 1 zu 0,5 bis 1 zu 3, insbesondere 1 zu 1 bis 1 zu 2, stehen können. Oxidationsfarbstoffvorprodukte vom Entwicklertyp und vom Kupplertyp werden besonders bevorzugt in bestimmten Kombinationen eingesetzt. Mit den als Kombination genannten Oxidationsfarbstoffvorprodukten und/oder deren physiologisch verträglichen Salzen können jedoch auch noch weitere Farbstoffvorprodukte kombiniert werden. Bevorzugte Mittel enthalten daher als Oxidationsfarbstoffprodukt zumindest eine der Kombinationen, ausgewählt aus p-Toluylendiamin / Resorcin; p-Toluylendiamin / 2-Methylresorcin; p-Toluylendiamin / 5-Amino-2-methylphenol; p-Toluylendiamin / 3-Aminophenol; p-Toluylendiamin / 2-(2,4-Diaminophenoxy)ethanol; p-Toluylendiamin / 1,3-Bis(2,4-diaminophenoxy)propan; p-Toluylendiamin / 1-Methoxy-2-amino-4-(2-hydroxyethylamino)benzol; p-Toluylendiamin / 2-Amino-3-hydroxypyridin; p-Toluylendiamin / 1-Naphthol; 2-(2-Hydroxyethyl)-p-phenylendiamin / Resorcin; 2-(2-Hydroxyethyl)-p-phenylendiamin / 2-Methylresorcin; 2-(2-Hydroxyethyl)-p-phenylendiamin / 5-Amino-2-methylphenol; 2-(2-Hydroxyethyl)-p-phenylendiamin / 3-Aminophenol; 2-(2-Hydroxyethyl)-p-phenylendiamin / 2-(2,4-Diaminophenoxy)ethanol; 2-(2-Hydroxyethyl)-p-phenylendiamin / 1,3-Bis-(2,4-diaminophenoxy)propan; 2-(2-Hydroxyethyl)-p-phenylendiamin / 1-Methoxy-2-amino-4-(2-hydroxyethylamino)benzol; 2-(2-Hydroxyethyl)-p-phenylendiamin / 2-Amino-3-hydroxypyridin; 2-(2-Hydroxyethyl)-p-phenylendiamin / 1-Naphthol; 2-Methoxymethyl-p-phenylendiamin / Resorcin; 2-Methoxymethyl-p-phenylendiamin / 2-Methylresorcin; 2-Methoxymethyl-p-phenylendiamin / 5-Amino-2-methylphenol; 2-Methoxymethyl-p-phenylendiamin / 3-Aminophenol; 2-Methoxymethyl-p-phenylendiamin / 2-(2,4-Diaminophenoxy)ethanol; 2-Methoxymethyl-p-phenylendiamin / 1,3-Bis(2,4-diaminophenoxy)propan; 2-Methoxymethyl-p-phenylendiamin / 1-Methoxy-2-amino-4-(2-hydroxyethylamino)benzol; 2-Methoxymethyl-p-phenylendiamin / 2-Amino-3-hydroxypyridin; 2-Methoxymethyl-p-phenylendiamin / 1-Naphthol; N-(4-Amino-3-methylphenyl)-N-[3-(1H-imidazol-1-yl)propyl]amin / Resorcin; N-(4-Amino-3-methylphenyl)-N-[3-(1H-imidazol-1-yl)propyl]amin / 2-Methylresorcin; N-(4-Amino-3-methylphenyl)-N-[3-(1H-imidazol-1-yl)propyl]amin / 5-Amino-2-methylphenol; N-(4-Amino-3-methylphenyl)-N-[3-(1H-imidazol-1-yl)propyl]amin / 3-Aminophenol; N-(4-Amino-3-methylphenyl)-N-[3-(1H-imidazol-1-yl)propyl]amin / 2-(2,4-Diaminophenoxy)ethanol; N-(4-Amino-3-methylphenyl)-N-[3-(1H-imidazol-1-yl)propyl]amin / 1,3-Bis(2,4-diaminophenoxy)-propan; N-(4-Amino-3-methylphenyl)-N-[3-(1H-imidazol-1-yl)propyl]amin / 1-Methoxy-2-amino-4-(2-hydroxyethylamino)benzol; N-(4-Amino-3-methylphenyl)-N-[3-(1H-imidazol-1-yl)propyl]amin / 2-Amino-3-hydroxypyridin; N-(4-Amino-3-methylphenyl)-N-[3-(1H-imidazol-1-yl)propyl]amin / 1-Naphthol; 4,5-Diamino-1-(2-hydroxyethyl)pyrazol / Resorcin; 4,5-Diamino-1-(2-hydroxyethyl)pyrazol / 2-Methylresorcin; 4,5-Diamino-1-(2-hydroxyethyl)pyrazol / 5-Amino-2-methylphenol; 4,5-Diamino-1-(2-hydroxyethyl)pyrazol / 3-Aminophenol; 4,5-Diamino-1-(2-hydroxyethyl)pyrazol / 2-(2,4-Diamino-phenoxy)ethanol; 4,5-Diamino-1-(2-hydroxyethyl)pyrazol / 1,3-Bis(2,4-diaminophenoxy)propan; 4,5-Diamino-1-(2-hydroxyethyl)pyrazol / 1-Methoxy-2-amino-4-(2-hydroxyethylamino)benzol; 4,5-Diamino-1-(2-hydroxyethyl)pyrazol / 2-Amino-3-hydroxypyridin sowie 4,5-Diamino-1-(2-hydroxyethyl)pyrazol / 1-Naphthol.

Im Zuge der Untersuchungen zu dieser Erfindung hat sich herausgestellt, dass durch die Verwendung von mindestens einem kationischen Polysiloxan der Formel (I) neben einer Verbesserung des Anwendungskomforts Nachteile der oxidativen Farbveränderung, wie zum Beispiel Schädigungen der Haarstruktur und -oberfläche durch Oxidationsmittel oder Alkalisierungsmittel, reduziert werden.

Weiterhin können die erfindungsgemäßen Mittel als farbverändernde Komponente mindestens einen direktziehenden Farbstoff enthalten. Dabei handelt sich um Farbstoffe, die direkt auf das Haar aufziehen und keinen oxidativen Prozess zur Ausbildung der Farbe benötigen. Direktziehende Farbstoffe sind üblicherweise Nitrophenylendiamine, Nitroaminophenole, Azofarbstoffe, Anthrachinone oder Indophenole. Die direktziehenden Farbstoffe werden jeweils bevorzugt in einer Menge von 0,001 bis 20 Gew.-%, bezogen auf die gesamte Anwendungszubereitung, eingesetzt. Die Gesamtmenge an direktziehenden Farbstoffen beträgt vorzugsweise höchstens 20 Gew.-%. Direktziehende Farbstoffe sind als anionische, kationische und nichtionische direktziehende Farbstoffe bekannt.

Bevorzugte anionische direktziehende Farbstoffe sind die unter den internationalen Bezeichnungen bzw. Handelsnamen Acid Yellow 1, Yellow 10, Acid Yellow 23, Acid Yellow 36, Acid Orange 7, Acid Red 33, Acid Red 52, Pigment Red 57:1, Acid Blue 7, Acid Green 50, Acid Violet 43, Acid Black 1, Acid Black 52, Bromphenolblau und Tetrabromphenolblau bekannten Verbindungen.

Bevorzugte kationische direktziehende Farbstoffe sind kationische Triphenylmethanfarbstoffe, wie beispielsweise Basic Blue 7, Basic Blue 26, Basic Violet 2 und Basic Violet 14, aromatische Systeme, die mit einer quaternären Stickstoffgruppe substituiert sind, wie beispielsweise Basic Yellow 57, Basic Red 76, Basic Blue 99, Basic Brown 16 und Basic Brown 17, sowie direktziehende Farbstoffe, die einen Heterocyclus enthalten, der mindestens ein quaternäres Stickstoffatom aufweist, insbesondere Basic Yellow 87, Basic Orange 31 und Basic Red 51. Die kationischen direktziehenden Farbstoffe, die unter dem Warenzeichen Arianor vertrieben werden, sind erfindungsgemäß ebenfalls besonders bevorzugte kationische direktziehende Farbstoffe.

Als nichtionische direktziehende Farbstoffe eignen sich insbesondere nichtionische Nitro- und Chinonfarbstoffe und neutrale Azofarbstoffe. Bevorzugte nichtionische direktziehende Farbstoffe sind die unter den internationalen Bezeichnungen bzw. Handelsnamen HC Yellow 2, HC Yellow 4, HC Yellow 5, HC Yellow 6, HC Yellow 12, HC Orange 1, Disperse Orange 3, HC Red 1, HC Red 3, HC Red 10, HC Red 11, HC Red 13, HC Red BN, HC Blue 2, HC Blue 11, HC Blue 12, Disperse Blue 3, HC Violet 1, Disperse Violet 1, Disperse Violet 4, Disperse Black 9 bekannten Verbindungen, sowie 1,4-Diamino-2-nitrobenzol, 2-Amino-4-nitrophenol, 1,4-Bis-(2-hydroxyethyl)-amino-2-nitrobenzol, 3-Nitro-4-(2-hydroxyethyl)aminophenol, 2-(2-Hydroxyethyl)amino-4,6-dinitrophenol, 4-[(2-Hydroxyethyl)amino]-3-nitro-1-methylbenzol, 1-Amino-4-(2-hydroxyethyl)amino-5-chlor-2-nitrobenzol, 4-Amino-3-nitrophenol, 1-(2'-Ureidoethyl)amino-4-nitrobenzol, 2-[(4-Amino-2-nitrophenyl)amino]benzoesäure, 6-Nitro-1,2,3,4-tetrahydrochinoxalin, 2-Hydroxy-1,4-naphthochinon, Pikraminsäure und deren Salze, 2-Amino-6-chloro-4-nitrophenol, 4-Ethylamino-3-nitrobenzoesäure und 2-Chlor-6-ethylamino-4-nitrophenol.

In einer weiteren Ausführungsform der vorliegenden Erfindung wird die farbverändernde Komponente aus Farbstoffvorstufen naturanaloger Farbstoffe ausgewählt.

Als Farbstoffvorstufen naturanaloger Farbstoffe werden bevorzugt solche Indole und Indoline eingesetzt, die mindestens zwei Gruppen ausgewählt aus Hydroxy- und/oder oder Aminogruppen, bevorzugt als Substituent am Sechsring, aufweisen. Diese Gruppen können weitere Substituenten tragen, z. B. in Form einer Veretherung oder Veresterung der Hydroxygruppe oder eine Alkylierung der Aminogruppe. In einer weiteren Ausführungsform enthalten die Färbemittel mindestens ein Indol- und/oder Indolinderivat. Erfindungsgemäße Zusammensetzungen, die Vorstufen naturanaloger Farbstoffe enthalten, werden bevorzugt als luftoxidative Färbemittel verwendet. In dieser Ausführungsform werden die besagten Zusammensetzungen folglich nicht mit einem zusätzlichen Oxidationsmittel versetzt. Die Farbstoffvorstufen naturanaloger Farbstoffe werden jeweils bevorzugt in einer Menge von 0,001 bis 5 Gew.-%, bezogen auf die gesamte Anwendungszubereitung, eingesetzt. Die Gesamtmenge an direktziehenden Farbstoffen beträgt vorzugsweise höchstens 3 Gew.-%.

Bevorzugte Derivate des Indolins sind 5,6-Dihydroxyindolin, N-Methyl-5,6-dihydroxyindolin, N-Ethyl-5,6-dihydroxyindolin, N-Propyl-5,6-dihydroxyindolin, N-Butyl-5,6-dihydroxyindolin sowie 5,6-Dihydroxyindolin-2-carbonsäure, bevorzugt 5,6-Dihydroxyindolin. Bevorzugte Derivate des Indols sind 5,6-Dihydroxyindol, N-Methyl-5,6-dihydroxyindol, N-Ethyl-5,6-dihydroxyindol, N-Propyl-5,6-dihydroxyindol, N-Butyl-5,6-dihydroxyindol, 5,6-Dihydroxyindol-2-carbonsäure, insbesondere 5,6-Dihydroxyindol.

Eine weitere Möglichkeit zur Farbgebung bietet die Verwendung von Färbemitteln, welche sogenannte Oxofarbstoffvorprodukte enthalten. Als erfindungsgemäße farbverändernde Komponente können daher in einer weiteren Ausführungsform auch Oxofarbstoffvorprodukte eingesetzt werden. Oxofarbstoffvorprodukte werden bevorzugt als Kombination aus mindestens einer Verbindung (Oxo1), die mindestens eine reaktive Carbonylgruppe enthält, mit mindestens einer Verbindung (Oxo2), ausgewählt aus C,H-aciden Verbindungen und/oder aus Verbindungen mit primärer oder sekundärer Aminogruppe oder Hydroxygruppe, eingesetzt.

Die vorgenannten Komponenten (Oxo1) und (Oxo2) sind im Allgemeinen selbst keine Farbstoffe, und eignen sich daher jede für sich genommen allein nicht zur Färbung keratinhaltiger Fasern. In Kombination bilden sie in dem nichtoxidativen Prozess der sogenannten Oxofärbung die eigentlichen Farbstoffe aus. Die resultierenden Färbungen besitzen teilweise Farbechtheiten auf der keratinhaltigen Faser, die mit denen der Oxidationsfärbung vergleichbar sind. Es ist jedoch erfindungsgemäß bevorzugt, wenn (Oxo2) nur unter C,H-aciden Verbindungen ausgewählt werden. Die voranstehend genannten Verbindungen (Oxo1) sowie (Oxo2) werden, wenn sie zum Einsatz kommen, jeweils vorzugsweise in einer Menge von 0,001 bis 10 Gew.-%, insbesondere von 0,01 bis 5 Gew.-%, jeweils bezogen auf Gesamtgewicht des anwendungsbereiten Mittels, verwendet.

Es ist nicht erforderlich, dass Oxidationsfarbstoffvorprodukte, direktziehende Farbstoffe, Oxofarbstoffvorprodukte oder naturanaloge Farbstoffe jeweils einheitliche Verbindungen darstellen. Vielmehr können, bedingt durch die Herstellungsverfahren für die einzelnen Farbstoffe, in untergeordneten Mengen noch weitere Komponenten enthalten sein, soweit diese nicht das Färbeergebnis nachteilig beeinflussen oder aus anderen Gründen, z. B. toxikologischen, ausgeschlossen werden müssen.

In einer weiteren Ausführungsform der vorliegenden Erfindung wird die farbverändernde Komponente aus Aufhellmitteln ausgewählt. Solche Aufhellmittel sind üblicherweise chemische Oxidationsmittel, mit deren Hilfe natürliche und künstliche Farbstoffe im Haar zerstört und das Haar damit gebleicht wird. Als Aufhellmittel kommen daher Persulfate, Peroxomonosulfate, Chlorite, Hypochlorite und insbesondere Wasserstoffperoxid oder dessen Anlagerungsprodukte an Harnstoff, Melamin sowie Natriumborat in Frage.

Der Aufhelleffekt kann auch neben einer Färbung gewünscht sein. Die erfindungsgemäßen Mittel können zusätzlich auch Blondier- und/oder Bleichmittel enthalten und somit als Mittel bereitgestellt werden, die gleichzeitig färbend und aufhellend wirken. Solche Mittel werden nachfolgend als "Färbemittel", als "aufhellende Färbemittel" oder als "Färbe- und Aufhellmittel" bezeichnet. Es ist daher bevorzugt, wenn das Mittel zusätzlich ein Oxidationsmittel, ausgewählt aus Wasserstoffperoxid sowie seinen festen Anlagerungsprodukten an organische und anorganische Verbindungen, enthält. Als feste Anlagerungsprodukte kommen erfindungsgemäß insbesondere die Anlagerungsprodukte an Harnstoff, Melamin, Polyvinylpyrrolidinon sowie Natriumborat in Frage.

Bevorzugt wird als Oxidationsmittel Wasserstoffperoxid eingesetzt. Bevorzugt beträgt die Menge an Wasserstoffperoxid im anwendungsbereiten Mittel 0,5 bis 12 Gew.-%, bevorzugt 0,8 bis 6 Gew.- %, jeweils bezogen auf das anwendungsbereite Mittel.

Solche Oxidationsmittelzubereitungen sind vorzugsweise wässrige, fließfähige Oxidationsmittelzubereitungen. Dabei sind bevorzugte Zubereitungen dadurch gekennzeichnet, dass die fließfähige Oxidationsmittelzubereitung, bezogen auf ihr Gewicht, 40 bis 90 Gew.-%, vorzugsweise 50 bis 85 Gew.-%, besonders bevorzugt 55 bis 80 Gew.-%, weiter bevorzugt 60 bis 77,5 Gew.-% und insbesondere 65 bis 75 Gew.-% Wasser enthält.

Soll helles oder aufgehelltes Haar bei der Behandlung eine Auffrischung des Aufhelleffekts erfahren, so kann es vorteilhaft sein, Wasserstoffperoxid und /oder eines seiner festen Anlagerungsprodukte in ein Shampoo oder ein ähnliches Behandlungsmittel einzuarbeiten. Damit wird jedoch in der Regel nur eine geringe Aufhellung erzielt. Für die starke Aufhellung sehr dunklen Haares ist der alleinige Einsatz von Wasserstoffperoxid oder dessen Anlagerungsprodukten an organische beziehungsweise anorganische Verbindungen oftmals nicht ausreichend. In diesen Fällen wird in der Regel eine Kombination aus Wasserstoffperoxid und anorganischen Persulfaten eingesetzt, woraus eine Steigerung des Aufhellvermögens der Mittel resultiert. Bevorzugte Persulfatsalz sind Ammoniumperoxodisulfat, Kaliumperoxodisulfat und Natriumperoxodisulfat.

Die Peroxodisulfatsalze können in einer Menge von 0,1 bis 25 Gew.-%, insbesondere in einer Menge von 0,5 bis 15 Gew.-%, bezogen auf das Gesamtgewicht des anwendungsbereiten Mittels, enthalten sein. Der Einsatz von Persulfatsalzen bzw. Peroxodisulfatsalzen erfolgt in der Regel in Form eines gegebenenfalls entstaubten Pulvers oder eines in Form gepressten Formkörpers.

Im Falle der oxidativen Färbungen kann die Entwicklung der Farbe grundsätzlich mit Luftsauerstoff erfolgen. Bevorzugt wird jedoch ein chemisches Oxidationsmittel eingesetzt, besonders dann, wenn neben der Färbung ein Aufhelleffekt an menschlichem Haar gewünscht ist. Erfindungsgemäß kann aber das Farbveränderungsmittel als Oxidationsfärbemittel auch zusammen mit einem Katalysator auf das Haar aufgebracht werden, der die Oxidation der Farbstoffvorprodukte, z.B. durch Luftsauerstoff, aktiviert. Solche Katalysatoren sind z. B. bestimmte Enzyme, lodide, Chinone oder Metallionen.

Bei einer Anwendung von zusätzlichen Oxidationsmitteln wird das eigentliche Färbe- und/oder Aufhellmittel zweckmäßigerweise unmittelbar vor der Anwendung durch Mischung einer erfindungsgemäßen Zubereitung, enthaltend in einem kosmetischen Träger mindestens ein Oxidationsfarbstoffvorprodukt, sowie einer Zubereitung, enthaltend das zusätzliche Oxidationsmittel, insbesondere Wasserstoffperoxid, hergestellt. Das erfindungsgemäße Mittel kann dabei das kationische Polysiloxan der Formel (I) in der Zubereitung mit Oxidationsfarbstoffvorprodukten und/oder in der Oxidationsmittelzubereitung enthalten. Bevorzugt enthält die Zubereitung mit Oxidationsfarbstoffvorprodukten das kationische Polysiloxan.

Wird eine starke Aufhellung gewünscht, so ist es erfindungsgemäß bevorzugt, wenn zusätzlich eine Blondierzubereitung, enthaltend mindestens ein anorganisches, der Oxidationsmittelzubereitung vor Vermischung mit der erfindungsgemäßen Färbezubereitung beigemischt wird.

Weiterhin hat es sich als vorteilhaft erweisen, wenn die Oxidationsmittelzubereitungen aus Oxidationsfärbemitteln und/oder Aufhellmitteln mindestens einen Stabilisator oder Komplexbildner enthalten. Gebräuchliche und im Rahmen der vorliegenden Erfindung bevorzugte Chelatkomplexbildner sind beispielsweise Polycarbonsäuren, stickstoffhaltige Mono- oder Polycarbonsäuren, insbesondere Ethylendiamintetraessigsäure (EDTA), Ethylendiamindibernsteinsäure (EDDS) und Nitrilotriessigsäure (NTA), geminale Diphosphonsäuren, insbesondere 1-Hydroxyethan-1,1-diphosphonsäure (HEDP), Aminophosphonsäuren wie Ethylendiamintetra(methylenphosphonsäure) (EDTMP) und Diethylentriaminpenta(methylenphosphonsäure) (DTPMP), Phosphonopolycarbonsäuren wie 2-Phosphonobutan-1,2,4-tricarbonsäure sowie Cyclodextrine, Alkalistannate (Natriumstannat), Alkalipyrophosphate (Tetranatriumpyrophosphat, Dinatriumpyrophosphat), Alkaliphosphate (Natriumphosphat), und Phosphorsäure. Erfindungsgemäß bevorzugt enthalten die Mittel Komplexbildner zu 0,01 bis 3 Gew.-%, bevorzugt 0,05 bis 1 Gew.-%, jeweils bezogen auf das Gesamtgewicht des erfindungsgemäßen Mittels.

Es ist erfindungsgemäß bevorzugt, dass das Mittel zusätzlich den genannten Farbveränderungsmitteln ein Oxidationsmittel, ausgewählt aus Wasserstoffperoxid sowie seinen festen Anlagerungsprodukten an organische und anorganische Verbindungen, enthält. Als feste Anlagerungsprodukte kommen erfindungsgemäß insbesondere die festen Anlagerungsprodukte an Harnstoff, Melamin, Polyvinylpyrrolidinon sowie Natriumborat in Frage.

Hinsichtlich der Mengen an Wasserstoffperoxid im anwendungsbereiten Mittel wird auf die voranstehenden, je nach Anwendungsgebiet bevorzugten Mengen verwiesen.

Vorzugsweise stellen die anwendungsbereiten Mittel fließfähige Zubereitungen dar. Bevorzugt enthalten die fließfähigen Zubereitungen zusätzlich als oberflächenaktive Substanz ein Emulgator bzw. ein Tensid, wobei oberflächenaktive Substanzen je nach Anwendungsgebiet als Tenside oder als Emulgatoren bezeichnet werden und aus anionischen, kationischen, amphoteren, zwitterionischen und weiteren nichtionischen Tensiden und Emulgatoren ausgewählt sind. Diese Stoffe werden nachfolgend ausführlich beschrieben.

Als anionische Tenside eignen sich in erfindungsgemäßen Zubereitungen alle für die Verwendung am menschlichen Körper geeigneten anionischen oberflächenaktiven Stoffe. Bevorzugte anionische Tenside sind Alkylsulfate, Alkylethersulfate, jeweils mit 10 bis 18 C-Atomen in der Alkylgruppe, und polyalkoxylierte Ethercarbonsäuren mit 10 bis 18 C-Atomen in der Alkylgruppe und bis zu 12 Glykolethergruppen im Molekül.

Als zwitterionische Tenside werden solche oberflächenaktiven Verbindungen bezeichnet, die im Molekül mindestens eine quartäre Ammoniumgruppe und mindestens eine Carboxylat-, Sulfonat-oder Sulfat-Gruppe tragen. Ein bevorzugtes zwitterionisches Tensid ist das unter der INCI-Bezeichnung Cocamidopropyl Betaine bekannte Fettsäureamid-Derivat.

In einer weiteren, bevorzugten Ausführungsform der vorliegenden Erfindung enthält das Mittel weiterhin mindestens ein amphoteres Tensid. Besonders bevorzugte amphotere Tenside werden unter der INCl-Bezeichnung Disodium Cocoamphodipropionate mit den Handelsnamen Miranol C2M SF conc. (Rhodia), Amphoterge K-2 (Lonza) und Monateric CEM-38 (Unichema) und Bezeichnung Disodium Cocoamphodiacetate mit den Handelsnamen Dehyton (Cognis), Miranol C2M (Rhodia) und Ampholak XCO 30 (Akzo Nobel) vermarktet.

Als besonders vorteilhaft haben sich außerdem oberflächenaktive Verbindungen vom Typ nichtionischer Tenside herausgestellt. Solche Verbindungen sind beispielsweise Anlagerungsprodukte von Polyethylenoxid an Fettalkohole, wobei Anlagerungsprodukte von Polyethylenoxid an Fettalkohole besonders bevorzugt sind, die einen durchschnittlichen Ethoxylierungsgrad von 10 bis 45, insbesondere von 12 bis 30 aufweisen, wie beispielsweise Steareth-20, Coceth-15, Oleth-20 oder auch Ceteareth-30, sowie Alkylpolyglykoside entsprechend der allgemeinen Formel RO-(Z)ₓ, wobei R für Alkyl, Z für Zucker sowie x für die Anzahl der Zuckereinheiten steht. Erfindungsgemäß bevorzugte Mittel enthalten als nichtionisches Tensid das Anlagerungsprodukt von Cocos-Fettalkohol an (Poly-)glucose, welches unter der INCl-Bezeichnung Coco-Glucoside bekannt ist.

Die anionischen, zwitterionischen, amphoteren und weiteren nichtionischen Tenside werden bevorzugt in einer Gesamtmenge von 0,1 bis 45 Gew.%, bevorzugt 1 bis 30 Gew.% und besonders bevorzugt von 1 bis 20 Gew.%, bezogen auf die Gesamtmenge des anwendungsbereiten Mittels, eingesetzt.

Erfindungsgemäß bevorzugt sind ebenfalls kationische Tenside vom Typ der quartären Ammoniumverbindungen, der Esterquats und der Amidoamine. Die kationischen Tenside sind in den erfindungsgemäß verwendeten Mitteln bevorzugt in Mengen von 0,05 bis 10 Gew.-%, bezogen auf das gesamte Mittel, enthalten. Mengen von 0,1 bis 5 Gew.-% sind besonders bevorzugt.

Es hat sich erfindungsgemäß als vorteilhaft erwiesen, wenn Mittel mindestens ein Verdickungsmittel enthalten. Bezüglich dieser Verdickungsmittel bestehen keine prinzipiellen Einschränkungen. Es können sowohl organische als auch rein anorganische Verdickungsmittel zum Einsatz kommen. Bevorzugt handelt es sich bei dem Verdickungsmittel um ein anionisches, synthetisches Polymer. Bevorzugte anionische Polymere sind Acrylsäure- und/oder Methacrylsäure-Polymere oder - Copolymerisate, die in den erfindungsgemäßen Mitteln bevorzugt in einer Menge von 0,1 bis 10 Gew.-%, besonders bevorzugt von 1 bis 5 Gew.-%, jeweils bezogen auf das Gewicht des Mittels, enthalten sind. Weiterhin bevorzugt handelt es sich bei dem Verdickungsmittel um ein kationisches synthetisches Polymer. Bevorzugte kationische Gruppen sind quartäre Ammoniumgruppen. Insbesondere solche Polymere, bei denen die quartäre Ammoniumgruppe über eine C₁-C₄-Kohlenwasserstoffgruppe an eine aus Acrylsäure, Methacrylsäure oder deren Derivaten aufgebaute Polymerhauptkette gebunden sind, haben sich als besonders geeignet erwiesen. Solche Polymere können auch als Copolymere mit nichtionogene Monomereinheiten, bevorzugt Acrylamid, Methacrylamid, Acrylsäure-C₁-C₄-Alkylester und Methacrylsäure-C₁-C₄-Alkylester, eingesetzt werden. Nichtionische, vollsynthetische Polymere, wie beispielsweise Polyvinylalkohol oder Polyvinylpyrrolidinon, sind ebenfalls als erfindungsgemäße Verdickungsmittel einsetzbar. Weiterhin bevorzugt werden natürlich vorkommende, gegebenenfalls modifizierte Verdickungsmittel eingesetzt. Dazu zählen z.B. Guargums, Skleroglucangums oder Xanthangums, pflanzliche Gums, wie Gummi arabicum, Ghatti-Gummi, Karaya-Gummi, Tragant-Gummi, Carrageen-Gummi, Agar-Agar, Johannisbrotkernmehl, Pektine, Alginate, Stärke, Stärke-Fraktionen und Derivate wie Amylose, Amylopektin und Dextrine, Cellulosederivate, wie beispielsweise Methylcellulose, Carboxyalkylcellulosen und Hydroxyalkylcellulosen. Als anorganische Verdickungsmittel haben sich Schichtsilikate (polymere, kristalline Natriumdisilicate) als besonders geeignet im Sinne der Erfindung erwiesen. Insbesondere Tone, vorzugsweise Magnesium Aluminium Silicate, wie beispielsweise Bentonit, besonders Smektite, wie Montmorillonit oder Hectorit, die gegebenenfalls auch geeignet modifiziert sein können, und synthetische Schichtsilikate sind bevorzugt.

Ferner können die erfindungsgemäßen Mittel weitere Wirk-, Hilfs- und Zusatzstoffe enthalten, wie beispielsweise nichtionische Polymere, kationische Polymere, zwitterionische und amphotere Polymere, anionische Polymere, Strukturanten wie Glucose, Maleinsäure und Milchsäure, haarkonditionierende Verbindungen wie Phospholipide, faserstrukturverbessernde Wirkstoffe, insbesondere Mono-, Di- und Oligosaccharide wie beispielsweise Glucose, Galactose, Fructose, Fruchtzucker und Lactose, Entschäumer wie Silikone, Farbstoffe zum Anfärben des Mittels, Antischuppenwirkstoffe, Wirkstoffe wie Aminosäuren, Oligopeptide und Proteinhydrolysate, Polyphenole und (Pseudo)Ceramide, Panthenol, Pantothensäure, Pantolacton, Allantoin, Pyrrolidinoncarbonsäuren und deren Salze sowie Bisabolol, Vitamine, Provitamine und Vitaminvorstufen, insbesondere solche der Gruppen A, B₃, B₅, B₆, C, E, F und H, Pflanzenextrakte, Fette und Wachse wie Bienenwachs, Montanwachs und Paraffine, Quell- und Penetrationsstoffe wie Glycerin, Propylenglykolmonoethylether, Carbonate, Hydrogencarbonate, Guanidine, Harnstoffe sowie primäre, sekundäre und tertiäre Phosphate, Trübungsmittel wie Latex, Styrol/PVP- und Styrol/Acrylamid-Copolymere, Perlglanzmittel wie Ethylenglykolmono- und -distearat sowie PEG-3-distearat, Pigmente, Treibmittel wie Propan-Butan-Gemische, N₂O Dimethylether, CO₂ und Luft und Antioxidantien.

Die Auswahl dieser weiteren Stoffe wird der Fachmann gemäß der gewünschten Eigenschaften der Mittel treffen. Bezüglich weiterer fakultativer Komponenten sowie der eingesetzten Mengen dieser Komponenten wird ausdrücklich auf die dem Fachmann bekannten einschlägigen Handbücher, z. B. Kh. Schrader, Grundlagen und Rezepturen der Kosmetika, 2. Auflage, Hüthig Buch Verlag, Heidelberg, 1989, verwiesen.

Erfindungsgemäß bevorzugte Mittel zur oxidativen Farbveränderung der Haare sind dadurch gekennzeichnet, dass sie einen alkalischen pH-Wert besitzen. Eine weitere bevorzugte Ausführungsform der vorliegenden Erfindung besteht darin, dass das anwendungsbereite Mittel einen pH-Wert zwischen 8,0 und 12,0, bevorzugt zwischen 8,5 und 11,5, insbesondere bevorzugt zwischen 9,0 und 11,0 besitzt. Bei den pH-Werten im Sinne der vorliegenden Erfindung handelt es sich um pH-Werte, die bei einer Temperatur von 22°C gemessen wurden. Üblicherweise wird der pH-Wert mit pH-Stellmitteln eingestellt. Zur Einstellung des pH-Werts sind dem Fachmann in der Kosmetik gängige Acidifizierungs- und Alkalisierungsmittel geläufig. Die zur Einstellung des pH-Wertes verwendbaren Alkalisierungsmittel werden typischerweise gewählt aus anorganischen Salzen, insbesondere der Alkali- und Erdalkalimetalle, organischen Alkalisierungsmitteln, insbesondere Aminen, basische Aminosäuren und Alkanolaminen, und Ammoniak. Erfindungsgemäß bevorzugte Acidifizierungsmittel sind Genuss-Säuren, wie beispielsweise Zitronensäure, Essigsäure, Äpfelsäure oder Weinsäure, sowie verdünnte Mineralsäuren. Erfindungsgemäß bevorzugte Alkalisierungsmittel sind ausgewählt aus der Gruppe, die gebildet wird aus Natriumhydroxid, Kaliumhydroxid, Calciumhydroxid, Bariumhydroxid, Natriumphosphat, Kaliumphosphat, Natriumsilicat, Kaliumsilicat, Natriumcarbonat, Kaliumcarbonat, 2-Aminoethan-1-ol, Triethanolamin, Ammoniak, 1-Aminopropan-2-ol und 2-Amino-2-methylpropan-1-ol.

Die Konfektionierung der erfindungsgemäßen Farbveränderungsmittel unterliegt prinzipiell keinerlei Beschränkungen. Üblicherweise werden die erfindungsgemäßen Mittel als 1-Komponentenmittel konfektioniert, die gegebenenfalls unmittelbar vor der Anwendung mit einer zweiten Zubereitung, enthaltend beispielsweise ein Oxidationsmittel, vermischt werden. Es hat sich aber in einigen Fällen auch als bevorzugt erwiesen, wenn das Produkt als 2-Komponentenmittel konfektioniert ist. Im Rahmen einer bevorzugten Ausführungsform werden die erfindungsgemäßen Mittel daher derart konfektioniert, dass eine der erfindungswesentlichen Komponenten separat verpackt ist. Dabei spielt es erfindungsgemäß zunächst keine Rolle, welche der erfindungsgemäßen Komponenten separat verpackt wird; es kann aber bevorzugt sein, die Zubereitung, die zusätzlich mindestens ein kationisches Polysiloxan der Formel (I) enthält, bis zur Anwendung separat zu verpacken.

Erfindungsgemäß bevorzugte Mittel sind dadurch gekennzeichnet, dass das Mittel unmittelbar vor der Anwendung durch Vermischen mindestens zweier Zubereitungen hergestellt wird, wobei die mindestens zwei Zubereitungen in mindestens zwei getrennt konfektionierten Containern bereitgestellt werden, und wobei ein Container ein Färbemittel enthält, welches in einem kosmetischen Träger mindestens ein Oxidationsfarbstoffvorprodukt und zusätzlich mindestens kationisches Polysiloxan der Formel (I) enthält, und ein weiterer Container eine Oxidationsmittelzubereitung, enthaltend mindestens ein Oxidationsmittel, enthält. Das Färbemittel besitzt dabei bevorzugt einen pH-Wert zwischen 8,0 und 12,0, bevorzugt zwischen 9,0 und 11,0.

Ein weiterer Gegenstand der vorliegenden Erfindung ist ein Verfahren zur Farb- und/oder Formveränderung keratinischer Fasern, bei dem ein erfindungsgemäßes Mittel gemäß obiger Vorgaben auf das Haar aufgetragen wird, für eine Einwirkzeit von 2 bis 45 min, bevorzugt von 15 bis 30 min auf dem Haar belassen wird, und anschließend das Haar ausgespült wird.

Eine bevorzugte Ausführungsform des erfindungsgemäßen Verfahrens zur Farbveränderung keratinischer Fasern liegt vor, wenn eine Zusammensetzung in einem kosmetischen Träger, enthaltend mindestens ein Oxidationsfarbstoffvorprodukt und zusätzlich mindestens kationisches Polysiloxan der Formel (I), mit einer Oxidationsmittelzubereitung, enthaltend Wasserstoffperoxid, zu einer homogenen Zusammensetzung vermischt, diese auf das Haar aufgebracht wird, für eine Einwirkzeit von 2 bis 45 min, bevorzugt von 5 bis 30 min auf dem Haar belassen wird, und anschließend das Haar ausgespült wird. Die Anwendungstemperaturen bei der erfindungsgemäßen Farbveränderung keratinischer Fasern können in einem Bereich zwischen 15 und 45°C liegen. Nach einer Einwirkungszeit wird das Haarfärbemittel durch Ausspülen von dem zu färbenden Haar entfernt. Das Nachwaschen mit einem Shampoo kann entfallen, wenn ein stark tensidhaltiger Träger, z. B. ein Färbeshampoo, verwendet wurde.

Bezüglich weiterer bevorzugter Ausführungsformen der erfindungsgemäßen Verfahren gilt mutatis mutandis das zu den erfindungsgemäßen Mitteln Gesagte.

Wie bereits erwähnt, können die erfindungsgemäßen Mittel auch direkt vor der Anwendung aus zwei oder mehreren getrennt verpackten Zubereitungen hergestellt werden. Dies bietet sich insbesondere zur Trennung inkompatibler Inhaltsstoffe an, um eine vorzeitige Reaktion zu vermeiden. Das anwendungsbereite Mittel wird bei solchen Systemen vom Anwender oder Verbraucher direkt vor der Anwendung durch Vermischen der Komponenten hergestellt. Ein Färbe- und/oder Aufhellungsmittel, bei dem die Oxidationsfarbstoffvorprodukte zunächst getrennt von der Oxidationsmittelzubereitung, enthaltend bevorzugt Wasserstoffperoxid, vorliegen, ist dabei bevorzugt.

Ein weiterer Gegenstand der vorliegenden Erfindung ist daher eine Mehrkomponentenverpackungseinheit (Kit-of-Parts), enthaltend mindestens zwei getrennt voneinander konfektionierte Container, wobei ein Container in einem kosmetischen Träger mindestens eine farbverändernde Verbindung und zusätzlich mindestens ein kationisches Polysiloxan der Formel (I) enthält, und ein Container eine Oxidationsmittelzusammensetzung, enthaltend mindestens ein chemisches Oxidationsmittel, insbesondere Wasserstoffperoxid, enthält.

Eine bevorzugte Ausführungsform dieses Gegenstand der vorliegenden Erfindung ist eine Mehrkomponentenverpackungseinheit (Kit-of-Parts), enthaltend mindestens zwei getrennt voneinander konfektionierte Container, wobei ein Container eine Färbemischung in einem kosmetischen Träger, enthaltend mindestens eine farbgebende Komponente, insbesondere mindestens ein Oxidationsfarbstoffvorprodukt, sowie zusätzlich mindestens ein kationisches Polysiloxan der Formel (I), enthält und ein zweiter Container eine Oxidationsmittelzusammensetzung, enthaltend mindestens ein chemisches Oxidationsmittel, insbesondere Wasserstoffperoxid, enthält.

Wird eine besonders starke Aufhellwirkung durch Einsatz von Persulfatsalzen bzw. Peroxodisulfatsalzen gewünscht, ist es erfindungsgemäß bevorzugt, diese der erfindungsgemäßen Mehrkomponentenverpackungseinheit (Kit-of-Parts) in Form eines gegebenenfalls entstaubten Pulvers oder eines in Form gepressten Formkörpers als separat verpackte, zusätzliche Komponente beizufügen.

Bevorzugt enthält die Mehrkomponentenverpackungseinheit (Kit-of-Parts) zusätzlich eine Gebrauchsanleitung. Darüber hinaus kann es bevorzugt sein, wenn weiterhin eine Anmischhilfe, wie beispielsweise eine Schale, eine Applikationshilfe, wie beispielsweise ein Kamm oder ein Pinsel, und/oder eine persönliche Schutzausrüstung, wie beispielsweise Einweg-Handschuhe dem Kit beigefügt ist. Bezüglich weiterer bevorzugter Ausführungsformen der Mehrkomponentenverpackungseinheit (Kit-of-Parts) gilt mutatis mutandis das zu den erfindungsgemäßen Mitteln Gesagte.

Ein weiterer Gegenstand der Erfindung ist die Verwendung der Mittel des ersten Erfindungsgegenstandes zur Verringerung der Schädigung der Haarstruktur bei der oxidativen Farbveränderung menschlicher Haare.

Ein weiterer Gegenstand der Erfindung ist schließlich die Verwendung der Mittel des ersten Erfindungsgegenstandes zur Pflege und zur Verbesserung der Naßkämmbarkeit, der Geschmeidigkeit und des Glanzes bei der oxidativen Farbveränderung menschlicher Haare bei der oxidativen Farbveränderung menschlicher Haare.

Bezüglich weiterer bevorzugter Ausführungsformen der erfindungsgemäßen Verwendungen gilt mutatis mutandis das zu den erfindungsgemäßen Mitteln Gesagte. Beispiele
1) Färbecreme (Angaben in Gew.-%):

| **Rohstoff** | **E1** |
|---|---|
| Lanette D [1] | 6,60 |
| Lorol techn. [2] | 2,40 |
| Eumulgin B 2 [3] | 0,60 |
| Eumulgin B 1 [4] | 0,60 |
| Lamesoft PO 65 [5] | 2,00 |
| Akypo Soft 45HP [6] | 10,00 |
| Texapon K 14 S Special, 70 % [7] | 2,80 |
| Produkt W 37194 [8] | 0,75 |
| p-Toluylendiaminsulfat | 0,20 |
| 2-Amino-6-chlor-4-nitrophenol | 0,10 |
| 5-Amino-2-methylphenol | 0,10 |
| 2,7-Dihydroxynaphthalin | 0,15 |
| Ammoniumsulfat, techn. rein | 0,90 |
| Natriumsulfit, wasserfrei, 96% | 0,40 |
| Natriumhydroxid, 45% | 0,6 |
| Natriumsilikat 40/42 | 0,50 |
| Abil T Quat 60 [*] | 3,00 |
| Ammoniak, 25 % | 6,50 |
| Parfum | qs |
| Wasser, entsalzt | add 100 |

2) Entwicklerzubereitung **EW**:

| **Rohstoff** | **Gew.-%** |
|---|---|
| Dipicolinsäure | 0,10 |
| Dinatriumpyrophosphat | 0,03 |
| HEDP, 60% | 1,50 |
| Texapon NSO [9] | 2,00 |
| Dow Corning DB 110 A [10] | 0,07 |
| Aculyn 33A [11] | 15,00 |
| Ammoniak, 25 % | 0,65 |
| Wasserstoffperoxid, 50 % | 12,00 |
| Wasser, vollentsalzt | ad 100 |

Rohstoffe: [1] INCl-Bezeichnung: Cetearyl alcohol (Cognis); [2] INCl-Bezeichnung: Coconut alcohol (Cognis); [3] INCl-Bezeichnung: Ceteareth-20 (Cognis); [4] INCl-Bezeichnung: Ceteareth-12 (Cognis); [5] INCl-Bezeichnung: Coco-Glucoside, Glyceryl Oleate, Aqua (Cognis); [6] ca. 21 %, INCl-Bezeichnung: Sodium Laureth-6 Carboxylate, Aqua (KAO); [7] ca. 70 %, INCl-Bezeichnung: Sodium Myreth Sulfate, Aqua (Cognis); [8] ca. 20 %, INCl-Bezeichnung: Acrylamidopropyltrimonium Chloride/Acrylates Copolymer, Aqua (Stockhausen); [9] ca. 28%, INCl-Bezeichnung: Sodium Laureth Sulfate (Cognis); [10] ca. 28% in Wasser; INCl-Bezeichnung: Acrylates Copolymer (Rohm & Haas); [11] INCl-Bezeichnung: Dimethicon (Dow Corning); [*] INCl-Bezeichnung: Silicone Quaternium-22 (Evonik).
3) Ausfärbungen:
Die Färbecreme E1 wurde vor der Anwendung mit einer Entwicklerlösung EW im Gewichtsverhältnis von 1:1 versetzt und innig vermischt (pH 9,5). Pro Gramm Haar (europäisches Humanhaar, Alkinco 6634, #10/2003, A9) wurden 4 g des frisch hergestellten, anwendungsbereiten Färbemittels aufgetragen. Die Einwirkzeit betrug 30 min bei 35°C für das Färbemittel. Danach wurden die Strähnen 30 s lang mit warmem Wasser ausgespült und luftgetrocknet. Die gefärbten Strähnen zeichneten sich durch glänzende Farben und einen angenehmen Griff aus.
4) Aufhellendes Haarpflegeshampoo:

| | |
|---|---|
| Ethoxyliertes Fettalkoholsulfat (C₁₂-C₁₄, 2 EO; 70% AS) | 15,0 |
| Disodium Cocoamphodiacetate | 7,0 |
| Euperlan PK 3000 AM [a] | 2,5 |
| Coconut Glycerides + 7,3 EO | 0,5 |
| Cutina HR [b] | 0,1 |
| D-Panthenol | 0,5 |
| Abil T Quat 60 | 0,5 |
| Zitronensäure | 0,5 |
| Wasserstoffperoxid | 0,2 |
| Salicylsäure | 0,2 |
| Trinatriumcitrat Dihydrat | 0,2 |
| Sodium Benzoate | 0,4 |
| Glycerin | 0,2 |
| NaCl | 1,0 |
| Parfum | qs |
| Wasser | ad 100 |

5) Aufhellender Rinse-off Conditioner:

| | |
|---|---|
| Cetearyl Alcohol | 2,5 |
| Eumulgin^{®} B2 | 0,5 |
| Genamin KDMP^{®} [c] | 3,0 |
| Cutina CP^{®} [d] | 0,5 |
| Natrosol 250 HR^{®} [e] | 0,5 |
| Timiron Super Silver^{®} [f] | 0,1 |
| Mannitol | 0,1 |
| Glycin | 0,2 |
| Methylparaben | 0,3 |
| Phenoxyethanol | 0,4 |
| D-Panthenol | 0,5 |
| Abil T Quat 60 | 1,0 |
| Wasserstoffperoxid | 0,4 |
| Citronensäure | 0,2 |
| Parfum | qs |
| Wasser | ad 100 |

Handelsprodukte: [a] INCl-Bezeichnung: Aqua, Glycol Distearate, Glycerin, Laureth-4, Cocamidopropyl Betaine, Formic Acid (Solids: 41-45%) (Cognis); [b] INCl-Bezeichnung: Hydrogenated Castor Oil (Cognis); [c] INCl-Bezeichnung: Behentrimonium Chloride (Cognis); [d] INCl-Bezeichnung: Cetyl Palmitate (Cognis); [e] INCl-Bezeichnung: Hydroxyethylcellulose (Hercules); [f] INCl-Bezeichnung: Mica; Cl 77891 (Merck).

## Patentansprüche

1. Mittel zur Farbveränderung von keratinischen Fasern, insbesondere menschlichen Haaren, enthaltend in einem kosmetischen Träger mindestens eine farbverändernde Komponente, **dadurch gekennzeichnet, dass** das Mittel zusätzlich mindestens ein kationisches Polysiloxan der Formel (I), 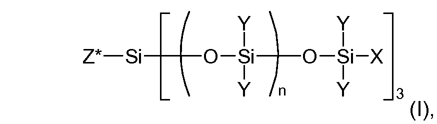 enthält, worin
X für gleiche oder verschiedene Ammoniumfunktionen tragende organische Reste,
Y für gleiche oder verschiedene Reste aus der Gruppe Alkyl, Aryl oder Alkylenaryl mit 1 bis 30 C-Atomen,
Z* für gleiche oder verschiedene Reste aus der Gruppe Alkyl, Aryl, oder Alkylenaryl mit 1 bis 30 C-Atomen,
und n für eine Zahl von 2 bis 200 stehen.

2. Mittel nach Anspruch 1, **dadurch gekennzeichnet, dass** das Polysiloxan der Formel (I) als Rest X eine Gruppe -R1-R2 enthält, worin R1 für gleiche oder verschiedene zweiwertige Reste, ausgewählt aus den Gruppen (a) bis (d), 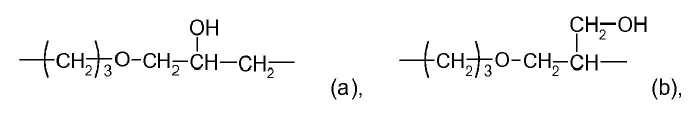 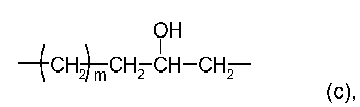 und/oder 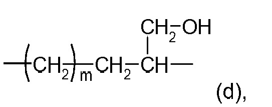 und R2 für eine Ammoniumfunktionen tragende Gruppe, ausgewählt aus den Gruppen (i) bis (iii), 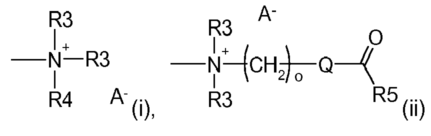 und/oder 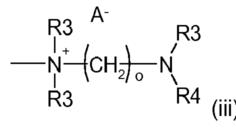 stehen, worin
R3, R4, R5 jeweils unabhängig voneinander für Wasserstoff oder einen Alkylrest mit 1 bis 30 C-Atomen,
Q für gleiche oder verschiedene Reste aus der Gruppe O und NR6,
R6 für gleiche oder verschiedene Reste aus Wasserstoff oder Alkyl mit 1 bis 6 C-Atomen,
m für eine Zahl von 2 bis 18 und O für eine Zahl von 2 bis 18 stehen,
wobei A⁻ gleiche oder verschiedene Gegenionen zu den positiven Ladungen an den quaternierten Stickstoffgruppen sind, ausgewählt aus anorganischen oder organischen Anionen der Säuren HA.

3. Mittel nach einem der Ansprüche 1 bis 2, **dadurch gekennzeichnet, dass** das Polysiloxan der Formel (I) als Rest X eine Gruppe -R1-R2 enthält, worin R1 für 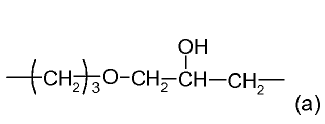 und R2 für 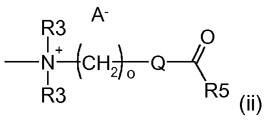 stehen, worin R3 für einen Alkylrest mit 1 bis 4 C-Atomen, R5 für einen Alkylrest mit 7 bis 23 C-Atomen, O für eine Zahl von 2 bis 5 und Q für die Gruppe NH stehen, sowie Y für gleiche oder verschiedene Alkyl-Reste mit 1 bis 4 C-Atomen, Z* für gleiche oder verschiedene Reste aus der Gruppe Alkyl, Aryl, oder Alkylenaryl mit 1 bis 30 C-Atomen und n für eine Zahl von 2 bis 200 stehen.

4. Mittel einem der Ansprüche 1 bis 3, **dadurch gekennzeichnet, dass** das Mittel ein oder mehrere Polysiloxane der Formel (I) in einer Gesamtmenge von 0,001 bis 10 Gew.-%, insbesondere von 0,01 bis 5,0 Gew.-%, jeweils bezogen auf das Gesamtgewicht des anwendungsbereiten Mittels, enthält.

5. Mittel nach einem der Ansprüche 1 bis 4, **dadurch gekennzeichnet, dass** das Mittel als farbverändernde Komponente mindestens ein Oxidationsfarbstoffvorprodukt und/oder mindestens einen direktziehenden Farbstoff und/oder mindestens einer Vorstufe naturanaloger Farbstoffe und/oder mindestens Oxofarbstoffvorprodukte und/oder mindestens ein Aufhellmittel enthält.

6. Mittel nach Anspruch 5, **dadurch gekennzeichnet, dass** es als farbverändernde Komponente mindestens ein Oxidationsfarbstoffvorprodukt enthält.

7. Mittel nach einem der Ansprüche 1 bis 6, **dadurch gekennzeichnet, dass** das Mittel zusätzlich ein Oxidationsmittel, ausgewählt aus Wasserstoffperoxid sowie seinen festen Anlagerungsprodukten an organische und anorganische Verbindungen, enthält.

8. Verwendung eines Mittels gemäß einem der Ansprüche 1 bis 7 zur Pflege und zur Verbesserung der Naßkämmbarkeit, der Geschmeidigkeit und des Glanzes bei der oxidativen Farbveränderung menschlicher Haare.

9. Verwendung eines Mittels gemäß einem der Ansprüche 1 bis 7 zur Verringerung der Schädigung der Haarstruktur bei der oxidativen Farbveränderung menschlicher Haare.

10. Kit-of-Parts, enthaltend mindestens zwei getrennt konfektionierte Container, wobei ein Container eine farbverändernde Verbindung gemäß einem der Ansprüche 1 bis 7 enthält, und ein Container eine Oxidationsmittelzusammensetzung, enthaltend mindestens ein chemisches Oxidationsmittel, insbesondere Wasserstoffperoxid, enthält.
